# EUROPÄISCHE PATENTSCHRIFT

(11) **EP 1 195 140 B1**
(45) Veröffentlichungstag und Bekanntmachung des Hinweises auf die Patenterteilung: **18.10.2006**
(21) Anmeldenummer: 01123731.0
(22) Anmeldetag: 04.10.2001
(51) Int. Cl.: A61B 6/00

(54) **Röntgensystem**
X-ray system
Système à rayons X

(30) Priorität: 06.10.2000 DE 10049538
(43) Veröffentlichungstag der Anmeldung: 10.04.2002
(73) Patentinhaber: Philips Intellectual Property & Standards GmbH, 20099 Hamburg (DE); Koninklijke Philips Electronics N.V., 5621 BA Eindhoven (NL)
(72) Erfinder: Schwieker, Horst-Hartwig, Philips Corporate, 52064 Aachen (DE)
(74) Vertreter: Volmer, Georg

(56) Entgegenhaltungen:
- EP-A- 0 165 157
- EP-A- 0 877 538
- GB-A- 665 729
- US-A- 5 014 292
- PATENT ABSTRACTS OF JAPAN vol. 1999, no. 14, 22. Dezember 1999 (1999-12-22) & JP 11 244271 A (HITACHI MEDICAL CORP), 14. September 1999 (1999-09-14)

## Beschreibung

Die Erfindung betrifft ein Röntgensystem mit einem Patientenlagerungstisch und einem Strahlungsmodul mit Röntgenstrahler und Strahlungsempfänger, zur Erzeugung eines Röntgenbildes eines Patienten.

Es ist bekannt, dass fast jedes Organ eines Patienten einer Röntgenuntersuchung unterzogen werden kann. Zu diesem Zweck sind verschiedene Verfahren bekannt, zu denen zum Beispiel auch die Tomographie, die periphere und stationäre Angiographie, die Phlebographie usw. gehören. Unterschieden wird weiterhin zwischen der Aufnahme von einzelnen Bildern und der Aufnahme von Bewegungsabläufen, d.h. zusammenhängenden Bildsequenzen. Diese zahlreichen Varianten spiegeln sich in einer entsprechenden Vielfalt von verschiedenen Röntgensystemen wieder, die im allgemeinen oder bevorzugt für jeweils eine dieser Untersuchungsarten ausgelegt sind. Je nach Untersuchungsart muss mit dem Patientenlagerungstisch eine zur Aufnahme des zu untersuchenden Organs geeignete Haltung bzw. Lagerung des Patienten erzielt werden, und das Strahlungsmodul muss in die entsprechende Bildaufnahmeposition gebracht werden können. Schließlich muss das Strahlungsmodul auch für die Art der Bildaufnahme ausgelegt sein.

Da in Krankenhäusern im allgemeinen alle Arten von Röntgenuntersuchungen durchgeführt werden müssen, sind unterschiedliche Röntgensysteme erforderlich, die einen erheblichen Kostenfaktor darstellen.

Dieses Problem wird noch dadurch verschärft, dass mit dem Einsatz von neuen digitalen Flachdetektoren, die zwar relativ teuer sind, die auf Grund ihrer Kompaktheit und der gegenüber den herkömmlichen Bildwandlern vereinfachten (digitalen) Bildverarbeitung jedoch wesentliche Vorteile aufweisen, die Kosten der Röntgensysteme weiter ansteigen.

Aus der JP 11244271 A ist ein Röntgensystem bekannt, bei welchem ein C-Bogen mit einem Röntgenstrahler und einem Strahlungsempfänger über einen Arm verschiebebeweglich an der seitlichen Führungsschiene eines Patientenlagerungstisches befestigt ist. Der zwischen dem C- Bogen und dem Patientenlagerungstisch gelegene Arm weist ein Drehgelenk auf, welches ein Schwenken des C-Bogens um eine zum Patiententisch senkrechte Achse ermöglicht.

Des Weiteren ist aus der GB 665 729 A ein Röntgensystem bekannt, bei welchem ein Röntgenstrahler und ein Strahlungsempfänger mit einem zwischen ihnen befindlichen Patiententisch fest verbunden sind und der Tisch um 180° um eine horizontale Achse geschwenkt werden kann.

Eine der Erfindung zugrunde liegende Aufgabe besteht deshalb darin, ein Röntgensystem der eingangs genannten Art zu schaffen, das universell für eine wesentlich größere Anzahl von verschiedenen Röntgenuntersuchungen einsetzbar ist.

Eine weitere, der Erfindung zugrunde liegende Aufgabe besteht darin, den an sich erwünschten Einsatz neuer Flachdetektoren bzw. R&F (Radiographie & Fluoroskopie) - Digitalsysteme durch Schaffung eines Röntgensystems zu erleichtern, das trotz der hohen Kosten dieser Detektoren sehr wirtschaftlich betrieben werden kann.

Gelöst werden diese Aufgaben gemäß Anspruch 1 mit einem Röntgensystem mit einem Patientenlagerungstisch und einem Strahlungsmodul mit Röntgenstrahler und Strahlungsempfänger, bei dem das Strahlungsmodul einen an dem Patientenlagerungstisch befestigten Träger umfasst, an dem gegenüberliegend der Röntgenstrahler und der Strahlungsempfänger befestigt sind, und der so schwenkbar ist, dass der Röntgenstrahler und der Strahlungsempfänger aus einer Position über bzw. unter einer Tischplatte des Patientenlagerungstisches ("Obertischtechnik") in eine umgekehrte Position unter bzw. über der Tischplatte ("Untertischtechnik") geführt werden können.

Ein besonderer Vorteil dieser Lösung besteht darin, dass das erfindungsgemäße Röntgensystem in einfacher Weise entsprechend der Art des abzubildenden Organs und der vorzunehmenden Untersuchung eingestellt werden kann und somit universell für eine wesentlich größere Anzahl von verschiedenen Untersuchungen einsetzbar ist, als dies mit bekannten Systemen möglich ist, wobei zum Beispiel auch Bildaufnahmen mit Schrägdurchstrahlung durchgeführt werden können.

Die Unteransprüche haben vorteilhafte Weiterbildungen der Erfindung zum Inhalt.

Die Ausführungen gemäß den Ansprüchen 2 und 3 haben den Vorteil, dass eine kompakte konstruktive Einheit entsteht, die relativ einfach aufbaubar und einstellbar ist.

Mit den Ausführungen gemäß den Ansprüchen 5 und 6 wird die Verstellbarkeit des Röntgenstrahlers und des Strahlungsempfängers zusätzlich erweitert. Insbesondere in Kombination mit den Verstellbarkeiten des Patientenlagerungstisches gemäß den in den Ansprüchen 4, 8 und 9 beschriebenen Ausführungen wird ein nahezu jede Röntgenuntersuchung umfassendes Anwendungsspektrum geschaffen.

Mit der Ausführung gemäß Anspruch 7 können schließlich der Röntgenstrahler und der Strahlungsempfänger insbesondere bei lateralen Untersuchungen jeweils gesondert optimal eingestellt werden.

Weitere Einzelheiten, Merkmale und Vorteile der Erfindung ergeben sich aus der folgenden Beschreibung einer bevorzugten Ausführungsform anhand der Zeichnung. Es zeigt:

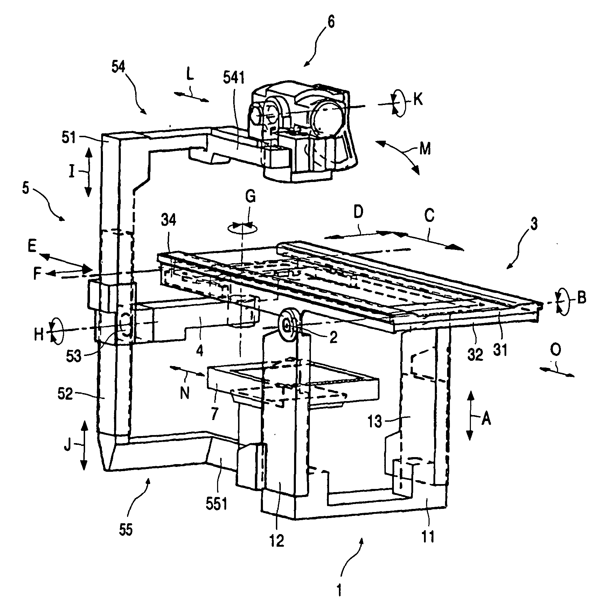

- Fig. 1: eine schematische dreidimensionale Ansicht eines erfindungsgemäßen Röntgensystems.

Das erfindungsgemäße Röntgensystem setzt sich aus einem Patientenlagerungstisch und einem Strahlungsmodul zusammen.

Der Patientenlagerungstisch umfasst ein im wesentlichen U-förmiges Gestell 1 mit einem Fuß 11 und zwei seitlichen, sich nach oben erstreckenden Streben 12, 13. Die beiden Streben sind gemäß Pfeil A teleskopartig ausfahrbar, so dass das Gestell 1 in seiner Höhe zum Beispiel in einem Bereich zwischen etwa 740 und 1220 mm verändert werden kann.

An den beiden oberen Enden der seitlichen Streben 12, 13 ist jeweils mittels eines Drehgelenkes 2 (nur eines davon ist dargestellt) eine Tischplatte 3 kippbar gelagert. Die Dimensionierung ist dabei so gewählt, dass die Tischplatte aus der in Figur 1 dargestellten waagerechten Stellung um eine horizontale erste, quer zur Tischplattenlängsrichtung verlaufende Achse (Pfeil B) um etwa 90 Grad nach vorne und um bis zu etwa 45 Grad nach hinten gekippt werden kann. Die Tischplatte 3 umfasst eine Auflageplatte 31 zur Auflage eines Patienten und einen Rahmen 32, an dem die Auflageplatte 31 so geführt ist, dass sie um etwa +/- 300 mm in Längsrichtung (Pfeil C) und um etwa +/- 80 m in Querrichtung (Pfeil D) verschoben werden kann. An dem Rahmen 32 ist ein Schlitten 34 (gestrichelt angedeutet) geführt, an dem das Strahlungsmodul befestigt ist und der in Längsrichtung der Tischplatte 3 gemäß Pfeil E um etwa 400 bis 700 mm verfahren werden kann.

Das Strahlungsmodul umfasst einen schwenkbaren Träger 4, 5, an dessen gegenüberliegenden Enden ein Röntgenstrahler 6 bzw. ein Strahlungsempfänger 7 (zum Beispiel ein Bildverstärker oder ein digitaler Flachdetektor mit Streustrahlenraster) angeordnet ist. Der Träger umfasst einen ersten, sich im wesentlichen parallel zur Ebene der Tischplatte 3 erstreckenden Arm 4, der mit einem ersten Ende so an dem Schlitten 34 gelagert ist, dass er - und damit das gesamte Strahlungsmodul - in Querrichtung der Tischplatte 3 gemäß Pfeil F um etwa 160 bis 320 mm verfahren werden kann. Aufgrund der Verfahrbarkeit des Schlittens 34 in Längsrichtung der Tischplatte 3 kann das gesamte Strahlungsmodul auch in dieser Richtung verschoben werden (Pfeil E).

Der erste Arm 4 ist mit seinem ersten Ende ferner um eine zur Tischplatte 3 senkrechte zweite Achse gemäß Pfeil G in der Weise schwenkbar an dem Schlitten 34 gelagert, dass er aus der in der Figur 1 gezeigten Stellung um einen Winkel von etwa 180 Grad nach rechts geschwenkt werden kann.

An dem zweiten Ende des ersten Arms 4 ist mittels einer Drehgelenkverbindung 53 drehbar ein zweiter Arm 5 (in Figur 1 vertikal verlaufend dargestellt) befestigt, der sich im wesentlichen in einer Ebene senkrecht zu der Ebene der Tischplatte 3 erstreckt und der mit einem Winkel von etwa +/- 180 Grad um die Längsachse (dritte Achse) des ersten Arms 4 gemäß Pfeil H, d.h. in einer zur Ebene der Tischplatte 3 senkrechten Ebene geschwenkt werden kann.

Der zweite Arm 5 umfasst einen in der Darstellung der Figur 1 oberen ersten Abschnitt 51, einen unteren zweiten Abschnitt 52, sowie die Drehgelenkverbindung 53, an der diese beiden Abschnitte so befestigt, geführt und ausgeformt sind, dass sie teleskopartig in ihrer Längsrichtung ineinander verfahren werden können, wie es durch die Pfeile I und J angedeutet ist. Der erste Abschnitt 51 kann dabei vorzugsweise bis auf eine Länge von etwa 800 mm und der zweite Abschnitt 52 bis auf eine Länge von etwa 600 mm gegenüber der Drehgelenkverbindung 53 ausgefahren werden.

Das Röntgensystem ist in dieser Gestaltung in der sogenannten Ober- und Untertischtechnik betreibbar. Dies bedeutet, dass der Röntgenstrahler 6 aus der Position oberhalb des Tisches 3 und der Strahlungsempfänger 7 aus der Position unterhalb des Tisches 3 ("Obertischtechnik") durch Schwenken des ersten Arms 4 um 90 Grad und des zweiten Arms 5 um 180 Grad in die jeweils umgekehrte, nämlich unterhalb bzw. oberhalb des Tisches liegende Position ("Untertischtechnik") geschwenkt werden kann. Dadurch ist auch die Aufnahme von Schrägbildern möglich, bei denen mit einer Schwenkbewegung des zweiten Arms 5 um wenige Grade eine Schrägdurchstrahlung des zu untersuchenden Bereiches vorgenommen wird. Weiterhin können durch entsprechendes Schwenken beider Arme auch freie Aufnahmen neben dem Tisch durchgeführt werden.

An das in der Figur 1 obere Ende des ersten Abschnitts 51 des zweiten Arms 5 schließt sich ein im wesentlichen L-förmig erstreckendes erstes Trägerstück 54 an, das in einer Ebene im wesentlichen parallel zu der dritten Achse (gemäß Pfeil H) liegt. Der Röntgenstrahler 6 ist entlang eines freien Schenkels 541 dieses ersten Trägerstücks 54 in Richtung des Pfeils L verschiebbar geführt. Der Röntgenstrahler 6 ist außerdem schwenkbar so befestigt, dass er um eine parallel zu der dritten Achse verlaufende vierte Achse gemäß Pfeil K mit einem Winkel von zum Beispiel etwa +/- 18 Grad geschwenkt werden kann.

Ein Kollimator des Röntgenstrahlers 6 kann schließlich entsprechend Pfeil M mit einem Winkel von etwa +/- 45 Grad um eine Zentralstrahlachse des Röntgenstrahlers geschwenkt werden. Dadurch kann der durch den Röntgenstrahl beleuchtete Bereich gedreht bzw. in seiner Form verändert werden, so dass eine gezielte Bestrahlung nur des zu untersuchenden Bereiches möglich ist, während alle anderen Bereiche abgeschattet sind.

An dem in Figur 1 unteren Ende des zweiten Abschnitts 52 des zweiten Arms 5 ist ein im wesentlichen L-förmig verlaufendes zweites Trägerstück 55 befestigt, das ebenfalls in einer Ebene im wesentlichen parallel zu der dritten Achse liegt. An einem freien Schenkel 551 dieses zweiten Trägerstücks 55 ist der Strahlungsempfänger 7 montiert, der entlang dieses Schenkels, d.h. in Richtung des Pfeils N, verschoben werden kann.

Die L-förmigen Trägerstücke 54, 55 ermöglichen auch die Durchführung von tomographischen Aufnahmen ("Schichtaufnahmetechnik"), wenn der Röntgenstrahlers 6 gemäß Pfeil L in einer Richtung und der Strahlungsempfängers 7 gemäß Pfeil N in einer dazu entgegengesetzten Richtung verschoben und der Röntgenstrahler 6 gleichzeitig um die vierte Achse gemäß Pfeil K geschwenkt wird, so dass er stets auf den Strahlungsempfänger gerichtet ist.

Die L-förmigen Trägerstücke 54, 55 ermöglichen weiterhin in optimaler Weise auch laterale Aufnahmen ("Lateraltischtechnik"), d.h. Aufnahmen in Querrichtung über den Tisch, indem ausgehend von der in Figur 1 gezeigten Stellung des Strahlungsmoduls zunächst der erste Arm 4 um 90 Grad nach rechts (hinten) und dann der zweite Arm 5 im Uhrzeigersinn um 90 Grad geschwenkt wird. Die Höhe des Strahlungskegels über dem Tisch kann dann durch Verschieben des Röntgenstrahlers 6 sowie des Strahlungsempfängers 7 entlang des jeweiligen freien Schenkels 541, 551 eingestellt werden.

Unter der Tischplatte 3 kann schließlich in bekannter Weise als weiterer Strahlungsempfänger (nicht dargestellt) ein Buckymodul (Rasterlade) vorgesehen sein, das in Richtung des Pfeils O verschiebbar ist und in bekannter Weise für fotografische Einzelaufnahmen verwendet werden kann.

Alle Bewegungen an dem Röntgensystem, die mit Bezug auf die Pfeile A bis O beschrieben wurden, werden vorzugsweise mit entsprechenden Elektromotoren und Getriebeeinheiten durchgeführt.

Mit dieser Geometrie und den beschriebenen Verstellbarkeiten im Hinblick auf ihre Art und ihren Umfang wird ein universelles Röntgensystem geschaffen, das für die Untersuchung von nahezu allen Organen mit den verschiedensten Verfahren, zum Beispiel auch in der Urologie und der Lithotripsie, geeignet ist.

Darüber hinaus ist das System sowohl zur Aufnahme von fotografischen Einzelbildern mit dem unter der Tischplatte 3 geführten Buckymodul, als auch zur Aufnahme von dynamischen, das heißt bewegten Bildern einsetzbar. Der an dem zweiten Trägerstück 55 des zweiten Arms 5 angeordnete Strahlungsempfänger 7 beinhaltet entweder einen bekannten Bildwandler oder einen digitalen Flachdetektor bzw. ein Digitalsystem, das für Radiologie und Fluoroskopie (R&F) vorgesehen ist. Da die Flachdetektoren auch zur Aufnahme von bis zu mehreren Bildern pro Sekunde geeignet sind, werden diese besonders bevorzugt in dem erfindungsgemäßen Röntgensystem eingesetzt, so dass das Buckymodul überflüssig ist. Durch die universelle Anwendbarkeit eines solchen Systems fällt auch der relativ hohe Preis dieser Detektoren nicht mehr so stark ins Gewicht.

Ein Röntgensystem kann modular aufgebaut sein und nach dem Baukastenprinzip in verschiedenen Ausbaustufen betrieben werden. In einer einfachsten Ausführung wird das Röntgensystem z.B. für urologische Untersuchungen verwendet. In diesem Fall braucht die Tischplatte 3 nur vertikal gemäß Pfeil A in der Höhe verstellt und gemäß Pfeil B gekippt zu werden, während im Hinblick auf das Strahlungsmodul nur die Bewegung des ersten Arms 4 in Längs- und Querrichtung des Tisches gemäß den Pfeilen E und F erforderlich ist. In einer etwas erweiterten Ausführung für urologische Untersuchungen ist unter der Tischplatte 3 zum Beispiel das Buckymodul geführt, und die Auflageplatte 31 ist auf dem Rahmen 32 in Längs- und Querrichtung gemäß den Pfeilen C und D verschiebbar.

Sofern mit einem solchen modularen Röntgensystem gleichzeitig Untersuchungen für die Lithotripsie durchgeführt werden sollen, ist das modulare Röntgensystem erfindungsgemäß erweiterbar, indem zusätzlich die Schwenkung des zweiten Arms 5 um die dritte Achse gemäß Pfeil H sowie die Verschiebung des Röntgenstrahlers 6 gemäß Pfeil I und die Verschiebung des Strahlungsempfängers 7 gemäß Pfeil J ermöglicht wird. Für diese Anwendung ist die obere Platte 31 vorzugsweise auf dem Rahmen 32 in Längs- und Querrichtung verschiebbar, um den Patienten entsprechend positionieren zu können.

Bei der in der Figur 1 gezeigten vollständigen Ausbaustufe ermöglichen die Längsverschiebung des Röntgenstrahlers 6 gemäß Pfeil L sowie das Kippen des Röntgenstrahlers 6 um die vierte Achse gemäß Pfeil K und die Längsverschiebung des Strahlungsempfängers 7 gemäß Pfeil N die Verwendung des Röntgensystems als Lungenstativ.

Die Modularität des Strahlungsmoduls und des Patientenlagerungstisches ermöglicht auch eine nachträgliche Anpassung des Röntgensystems an ein erweitertes Anwendungsspektrum, indem zum Beispiel einzelne oder mehrere der für die beschriebenen Bewegungen erforderlichen Komponenten nachträglich noch eingebaut bzw. ausgetauscht werden.

## Patentansprüche

1. Röntgensystem mit einem Patientenlagerungstisch und einem Strahlungsmodul mit Röntgenstrahler und Strahlungsempfänger, wobei das Strahlungsmodul einen an dem Patientenlagerungstisch (1, 3) befestigten Träger (4, 5, 51 bis 55) umfasst, an dem gegenüberliegend der Röntgenstrahler (6) und der Strahlungsempfänger (7) befestigt sind,
**dadurch gekennzeichnet,**
**dass** der Träger so schwenkbar ist, dass der Röntgenstrahler (6) und der Strahlungsempfänger (7) aus einer Position über bzw. unter einer Tischplatte (3) des Patientenlagerungstisches in eine umgekehrte Position unter bzw. über der Tischplatte (3) geführt werden können,
und **dass** der schwenkbare Träger einen ersten Arm (4) aufweist, der mit einem ersten Ende an der Tischplatte (3) gelagert ist, so dass er um eine durch die Tischplatte im wesentlichen senkrecht verlaufende zweite Achse schwenkbar ist.

2. Röntgensystem nach Anspruch 1,
**dadurch gekennzeichnet,**
**dass** der erste Arm (4) mit seinem ersten Ende an einem Rahmen (32) der Tischplatte (3) gelagert ist.

3. Röntgensystem nach Anspruch 2,
**dadurch gekennzeichnet,**
**dass** an dem zweiten Ende des ersten Arms (4) mittels einer Drehgelenkverbindung (53) ein zweiter Arm (5) befestigt ist, der den Röntgenstrahler (6) und den Strahlungsempfänger (7) trägt und der in einer zur Ebene der Tischplatte (3) im wesentlichen senkrechten Ebene schwenkbar ist.

4. Röntgensystem nach Anspruch 1,
**dadurch gekennzeichnet,**
**dass** die Tischplatte (3) mittels eines Drehgelenkes (2) um eine in ihrer Querrichtung verlaufende erste Achse kippbar an einem Gestell (1) gelagert ist.

5. Röntgensystem nach Anspruch 2,
**dadurch gekennzeichnet,**
**dass** an dem Rahmen (32) der Tischplatte (3) ein in dessen Längsrichtung verschiebbarer Schlitten (34) geführt ist, an dem der erste Arm (4) in Querrichtung des Tischplatte verschiebbar gelagert ist.

6. Röntgensystem nach Anspruch 3,
**dadurch gekennzeichnet,**
**dass** sich der zweite Arm (5) aus einem ersten Abschnitt (51) und einem zweiten Abschnitt (52) zusammensetzt, die an der Drehgelenkverbindung (53) so geführt sind, dass sie jeweils teleskopartig ineinander verfahren werden können.

7. Röntgensystem nach Anspruch 6,
**dadurch gekennzeichnet,**
**dass** an den der Drehgelenkverbindung (53) gegenüberliegenden Enden des ersten und zweiten Abschnitts (51, 52) jeweils ein erstes bzw. zweites, im wesentlichen L-förmiges Trägerstück (54, 55) angeordnet ist, die jeweils in einer Ebene im wesentlichen senkrecht zu dem zweiten Arm (5) liegen, wobei der Röntgenstrahler (6) bzw. der Strahlungsempfänger (7) entlang jeweils eines freien Schenkels (541, 551) der Trägerstücke verschiebbar befestigt ist.

8. Röntgensystem nach Anspruch 4,
**dadurch gekennzeichnet,**
**dass** die Tischplatte (3) eine Auflageplatte (31) und einen Rahmen (32) umfasst, wobei die Auflageplatte (31) so an dem Rahmen (32) geführt ist, dass sie in Längs- und Querrichtung verschiebbar ist.

9. Röntgensystem nach Anspruch 4,
**dadurch gekennzeichnet,**
**dass** das Gestell (1) in seiner Höhe verstellbar ist.

10. Röntgensystem nach einem der vorhergehenden Ansprüche,
**dadurch gekennzeichnet,**
**dass** das Strahlungsmodul und der Patientenlagerungstisch modular aufgebaut sind.

## Claims

1. An X-ray system which includes a patient table and a radiation module with an X-ray source and a radiation receiver, the radiation module comprising a carrier (4, 5, 51 to 55) attached to the patient table (1, 3) to which carrier the X-ray source (6) and the radiation receiver (7) are attached facing each other, **characterized in that** the carrier is pivotable in such a manner that the X-ray source (6) and the radiation receiver (7) can be moved from a position over and under a table top (3) of the patient table, respectively, to a reversed position under and over the table (3), respectively, and **in that** the pivotable carrier includes a first arm (4) which is journaled with a first end to the table top (3) so that it is pivotable about a second axis running in essence perpendicularly to the table top.

2. An X-ray system as claimed in claim 1, **characterized in that** the first end of the first arm (4) is journaled to a frame (32) of the table (3).

3. An X-ray system as claimed in claim 2, **characterized in that** a second arm (5) is attached to the second end of the first arm (4) by way of a pivot (53), said second arm carrying the X-ray source (6) and the radiation receiver (7) and being pivotable in a plane which extends essentially perpendicularly to the plane of the table top (3).

4. An X-ray system as claimed in claim 1, **characterized in that** the table top (3) is journaled on a frame (1) so as to be tiltable about a first axis, extending in its transverse direction, by way of a pivot (2).

5. An X-ray system as claimed in claim 2, **characterized in that** a carriage (34) is mounted on the frame (32) of the table top (3) so as to be slidable in the longitudinal direction thereof, the first arm (4) being journaled on said carriage (34) so as to be slidable in the transverse direction of the table top.

6. An X-ray system as claimed in claim 3, **characterized in that** the second arm (5) is composed of a first section (51) and a second section (52), said sections being mounted on the pivot (53) in such a manner that they can be moved telescopically one in the other.

7. An X-ray system as claimed in claim 6, **characterized in that** at opposite ends of the first and the second section (51, 52) on the pivot (53) there is arranged a first, essentially L-shaped carrier section and a second, essentially L-shaped carrier section (54, 55), each of said sections being situated in a plane which is essentially perpendicular to the second arm (5), the X-ray source (6) and the radiation receiver (7) being mounted so as to be slidable along a respective free limb (541, 551) of the carrier sections.

8. An X-ray system as claimed in claim 4, **characterized in that** the table (3) includes a supporting plate (31) and a frame (32), the supporting plate (31) being mounted on the frame (32) in such a manner that it is displaceable in longitudinal direction and transverse direction.

9. An X-ray system as claimed in claim 4, **characterized in that** the height of the frame (1) is adjustable.

10. An X-ray system as claimed in one of the preceding claims, **characterized in that** the radiation module and the patient table have a modular construction.

## Revendications

1. Système à rayons X avec une table d'examen de patient et un module de rayonnement avec un générateur de rayons X et un récepteur de rayons X, le module de rayonnement comprenant un support (4, 5, 51 à 55) fixé à la table d'examen de patient (1, 3) sur lequel sont fixés en face à face le générateur de rayons X (6) et le récepteur de rayons X (7),
**caractérisé en ce**
**que** le support peut pivoter de telle sorte que le générateur de rayons X (6) et le récepteur de rayons X (7) puissent être guidés à partir d'une position au-dessus ou en dessous d'un dessus de table (3) de la table d'examen de patient dans une position inverse en dessous ou au-dessus du dessus de table (3),
et **que** le support pivotant présente un premier bras (4) qui est logé avec une première extrémité sur le dessus de table (3) de telle sorte qu'il puisse pivoter autour d'un deuxième axe s'étendant dans une direction essentiellement perpendiculaire au dessus de la table.

2. Système à rayons X selon la revendication 1,
**caractérisé en ce**
**que** le premier bras (4) est logé avec sa première extrémité contre un cadre (32) du dessus de table (3).

3. Système à rayons X selon la revendication 2,
**caractérisé en ce**
**qu'**un deuxième bras (5) qui porte le générateur de rayons X (6) et le récepteur de rayons X (7) et qui peut pivoter dans un plan essentiellement perpendiculaire au plan du dessus de table (3) est fixé à la deuxième extrémité du premier bras (4) à l'aide d'une fixation par articulation rotative (53).

4. Système à rayons X selon la revendication 1,
**caractérisé en ce**
**que** le dessus de table (3) est logé à l'aide d'une articulation rotative (2) autour d'un premier axe s'étendant dans sa direction transversale pour basculer sur un châssis (1).

5. Système à rayons X selon la revendication 2,
**caractérisé en ce**
**qu'**un chariot (34) déplaçable dans sa direction longitudinale sur lequel est logé à coulissement le premier bras (4) dans la direction transversale du dessus de table est guidé sur le cadre (32) du dessus de table (3).

6. Système à rayons X selon la revendication 3,
**caractérisé en ce**
**que** le deuxième bras (5) se compose d'une première section (51) et d'une deuxième section (52) qui sont guidées sur la fixation par articulation rotative (53) de telle sorte qu'elles puissent pénétrer l'une dans l'autre de manière télescopique.

7. Système à rayons X selon la revendication 6,
**caractérisé en ce**
**que** des première ou deuxième pièces de support (54, 55) essentiellement en forme de L qui sont respectivement disposées dans un plan essentiellement perpendiculaire au deuxième bras (5) sont disposées aux extrémités opposées à la fixation par articulation rotative (53) des première ou deuxième sections (51, 52), le générateur de rayons X (6) ou le récepteur de rayons X (7) étant fixés à coulissement le long d'un côté libre respectif (541, 551) des pièces de support.

8. Système à rayons X selon la revendication 4,
**caractérisé en ce**
**que** le dessus de table (3) comprend une plaque d'appui (31) et un cadre (32), la plaque d'appui (31) étant guidée sur le cadre de telle manière qu'elle puisse coulisser en direction longitudinale et transversale.

9. Système à rayons X selon la revendication 4,
**caractérisé en ce**
**que** le bâti (1) est réglable en hauteur.

10. Système à rayons X selon l'une des revendications précédentes,
**caractérisé en ce**
**que** le module à rayons X et la table d'examen de patient sont de conception modulaire.
